# EUROPÄISCHE PATENTSCHRIFT

(11) **EP 3 557 276 B1**
(45) Veröffentlichungstag und Bekanntmachung des Hinweises auf die Patenterteilung: **03.07.2024**
(21) Anmeldenummer: 18167520.8
(22) Anmeldetag: 16.04.2018
(51) Int. Cl.: G01R 33/48, G01R 33/565, G01R 33/56

(54) **VERFAHREN UND MAGNETRESONANZTOMOGRAPHIESYSTEM ZUR ERZEUGUNG VON MAGNETRESONANZAUFNAHMEN INNERHALB UND AUSSERHALB DES B0-HOMOGENITÄTSVOLUMENS**
METHOD AND MAGNETIC RESONANCE TOMOGRAPHY SYSTEM FOR PRODUCING MAGNETIC RESONANCE IMAGES WITHIN AND OUTSIDE OF THE HOMOGENEOUS REGION OF THE B0-FIELD
PROCÉDÉ ET SYSTÈME D'IMAGERIE PAR RÉSONANCE MAGNÉTIQUE DESTINÉS À LA GÉNÉRATION D'IMAGES PAR RÉSONANCE MAGNÉTIQUE À L'INTERIEUR ET À L'EXTERIEUR DU VOLUME D'HOMOGÉNÉITÉ DU CHAMP MAGNÉTIQUE B0

(43) Veröffentlichungstag der Anmeldung: 23.10.2019
(73) Patentinhaber: Siemens Healthineers AG, 91301 Forchheim (DE)
(72) Erfinder: Popescu, Stefan, 91056 Erlangen (DE)
(74) Vertreter: Siemens Healthineers Patent Attorneys

(56) Entgegenhaltungen:
- WO-A1-2008/065389
- DE-A1-102012 213 696
- US-A1- 2012 265 050
- JEFFREY FESSLER: "Model-Based Image Reconstruction for MRI", IEEE SIGNAL PROCESSING MAGAZINE, IEEE SERVICE CENTER, PISCATAWAY, NJ, US, Bd. 27, Nr. 4, 1. Juli 2010 (2010-07-01), Seiten 81-89, XP011311323, ISSN: 1053-5888

## Beschreibung

Die Erfindung betrifft ein Verfahren zur Erzeugung von Magnetresonanzaufnahmen eines Untersuchungsobjekts in einem Magnetresonanztomographiesystem sowie ein Magnetresonanztomographiesystem zur Durchführung des Verfahrens. Mit Hilfe moderner bildgebender Verfahren werden häufig zwei- oder dreidimensionale Bilddaten oder Zeitserien von Bilddaten erzeugt, die zur Visualisierung vom Inneren eines Körpers genutzt werden können. Ein Untersuchungsobjekt kann dabei beispielsweise ein Patient oder Proband sein, beispielsweise bei einem Ganzkörperscan. Meist handelt es sich aber nur um einen Teil eines Patienten, wie beispielsweise den Brustkorb oder ein Organ, etc. Bildgebende Systeme, die auf einem Verfahren der Magnetresonanzmessung basieren, sogenannte Magnetresonanztomographen, haben sich durch vielfältige Anwendungen erfolgreich etabliert und bewährt. Bei dieser Art der Bildakquisition wird meist ein statisches Grundmagnetfeld B₀, das zur Anfangsausrichtung und Homogenisierung von zu untersuchenden magnetischen Dipolen dient, zur Ortsauflösung des bildgebenden Signals mit einem schnell geschalteten Magnetfeld, dem sogenannten Gradientenfeld, überlagert.

Zur Bestimmung von Materialeigenschaften eines abzubildenden Untersuchungsobjekts wird die Dephasierung bzw. Relaxationszeit nach einer Auslenkung der Magnetisierung aus der Anfangsausrichtung ermittelt, sodass verschiedene materialtypische Relaxationsmechanismen bzw. Relaxationszeiten identifiziert werden können. Die Auslenkung erfolgt meist durch eine Anzahl von HF-Pulsen und die Ortsauflösung beruht dabei auf einer zeitlich festgelegten Manipulation der ausgelenkten Magnetisierung mit Hilfe des Gradientenfeldes in einer sogenannten Messsequenz bzw. Ansteuersequenz, welche eine genaue zeitliche Abfolge von HF-Pulsen, der Änderung des Gradientenfeldes (durch Aussenden einer Schaltsequenz von Gradientenpulsen) sowie der Erfassung von Messwerten festlegt.

Typischerweise erfolgt eine Zuordnung zwischen gemessener Magnetisierung - aus der die erwähnten Materialeigenschaften abgeleitet werden können - und einer Ortskoordinate der gemessenen Magnetisierung im Ortsraum, in dem das Untersuchungsobjekt angeordnet ist, mit Hilfe eines Zwischenschritts. In diesem Zwischenschritt werden erfasste Magnetresonanzrohdaten in einem Frequenzraum erfasst und darauf basierend die Bilddaten rekonstruiert bzw. die Magnetresonanzrohdaten werden in einen Bildraum transformiert. In der Regel handelt es sich bei dem Frequenzraum um den sogenannten "k-Raum". Hierbei werden die Magnetresonanzrohdaten an Auslesepunkten im k-Raum angeordnet, wobei die Koordinaten des k-Raums als Funktion des Gradientenfeldes kodiert sind. Der Betrag der Magnetisierung (insbesondere der Quermagnetisierung, in einer Ebene quer zum vorbeschriebenen Grundmagnetfeld bestimmt) an einem bestimmten Ort des Untersuchungsobjekts kann aus den k-Raum-Daten mit Hilfe einer Fourier-Transformation ermittelt werden. Anders ausgedrückt, werden die k-Raum-Daten (Magnitude und Phase) benötigt, um eine Signalstärke des Signals und gegebenenfalls dessen Phase im Ortsraum zu berechnen.

Die Grundmagnetfelder B₀ von ca. 0,5 T und größer, welche in einem Magnetresonanztomographen herrschen können, werden mittels supraleitender Grundmagnetfeldmagneten erzeugt, die gewöhnlich mehrere supraleitende Spulen umfassen.

In der Regel werden die Spulen derart angeordnet, dass ein homogener Bereich des Magnetfelds eine sphärische Form aufweist, ein sogenanntes sphärisches Bildvolumen SIV.

In der Realität ist das Grundmagnetfeld B₀ aber nicht bzw. nicht überall homogen, sondern, weist vor allem je weiter es von einem Iso-Zentrum, d. h dem Zentrum eines Tunnels bzw.

Rings eines Magnetresonanztomographen entfernt ist, Inhomogenitäten auf.

Für eine erfolgreiche Aufnahme eines Untersuchungsobjekts, wird aber in der Regel vorausgesetzt, dass das Grundmagnetfeld B₀ in dem gesamten Aufnahmevolumen bzw. einem Sichtfeld (engl. Field of View), üblicherweise und auch alternativ als FoV bezeichnet, hinreichend stark und homogen ist, um möglichst exakte Messungen vornehmen zu können.

Das messbare Volumen bzw. Aufnahmevolumen ist somit aufgrund von physikalischen und technischen Bedingungen, wie z. B. der beschränkten Magnetfeldhomogenität und einer Nichtlinearität des Gradientenfelds, in alle drei Raumrichtungen, beschränkt. Dabei wird das Sichtfeld, auf ein Volumen beschränkt, in welchem die oben genannten physikalischen Merkmale innerhalb eines vorgegebenen Toleranzbereichs liegen (z.B. kann gefordert sein, dass die Homogenität des statischen Magnetfelds B₀ eine kleinere Abweichung als z. B. 20 ppm peak-to-peak oder weniger als 3 ppm Root mean square(RMS)aufweist) und daher eine ausreichend exakte Abbildung des zu untersuchenden Objekts mit üblichen Messsequenzen möglich ist. Daher entspricht das Sichtfeld normalerweise maximal dem sphärischen Bildvolumen SIV.

Um einen größeren Homogenitätsbereich zu erzielen und um somit das Sichtfeld zu erhöhen kann die Anzahl an supraleitenden Spulen erhöht werden. Dies geht jedoch mit steigenden Kosten und einer steigenden Baugröße eines Magnetresonanztomographen einher.

Inzwischen wurden weit geöffnete Ringtunnel- (engl. Wide Bore) Magnetresonanztomographiesysteme mit einem Ringtunneldurchmesser von 70 cm eingeführt. Diese Magnetresonanztomographiesysteme vereinen den hervorragenden Patientenzugang und -komfort eines offenen Tunnel- (engl. Open Bore) Magnetresonanztomographiesystems mit einer hochwertigen Bildgebung eines konventionell geschlossenen Tunnel- (engl. Closed Bore) Magnetresonanztomographiesystems. Aufgrund dieser Kombination sind die Wide Bore-Magnetresonanztomographiesysteme zu einem Standard in vielen Kliniken geworden. Nichtsdestotrotz hat sich die Größe des Bildvolumens bzw. des Sichtfelds mit einem Wide Bore-Magnetresonanztomographiesystem nicht verbessert. Der sogenannte Durchmesser DSV des sphärischen Bildvolumens SIV bleibt auch bei einem Magnetresonanztomographiesystem mit einer 70 cm Ringtunnelöffnung bei nur 55 cm. Dieser Durchmesser ist jedoch auch schon bei einem der herkömmlichen Magnetresonanztomographiesysteme mit einer Ringtunnelöffnung von nur 60 cm üblich. Das Sichtfeld ist dabei vor allem in x- und y-Richtung, d.h. senkrecht zu einer Längsachse des Ringtunnels eines Magnetresonanztomographen, erheblich geringer als das durch den Ringtunnel des Magnetresonanztomographen beschränkte Volumen.

In der EP 0244 724 A2 wird daher vorgeschlagen, ein MR-Bild in einer bestimmten Schichtposition aufzunehmen, in der das statische Magnetfeld am Rand des MR-Bildes ein vorbestimmtes Homogenitätskriterium erfüllt. Hierbei gibt es einige transversale Schichten, die an bestimmten Schichtpositionen entlang der Z-Achse angeordnet sind, die einen Durchmesser einer zugehörigen homogenen Scheibe aufweisen, welcher signifikant größer sein könnte als ein Durchmesser, der mittels einer herkömmlichen Rekonstruktion im sphärischen Bildvolumen SIV erwartet werden würde.

Dadurch erweitert dieses Verfahren jedoch nicht die Größe des sphärischen Bildgebungsvolumens SIV, sondern verwendet dagegen einfach ein Bildgebungsvolumenmodell, das eine sehr komplexe Außenkontur aufweist, anstatt des sphärischen Volumenmodells. Ein Nachteil dieses Verfahrens besteht darin, dass bei komplementären und verschachtelten Schichtpositionen entlang der Z-Achse, das Bildgebungsvolumen immer noch durch den Durchmesser DSV des sphärischen Bildvolumens SIV begrenzt ist.

Ein Beispiel eines Verfahrens zur Magnetresonanzbildgebung unter Anwendung einer Verzeichniskorrektur ist in der DE102012213696A1 offenbart. In der WO2008065389A1 wird ein Magnetresonanzgerät beschrieben, das mit inhomogenen Magnetfeldern arbeitet. Die US2012265050A1 offenbart ein kombiniertes CT-, MRT-, PET, SPECT- und Ultraschallgerät, welches ebenfalls mit inhomogenen Magnetfeldern arbeitet.

Es ist eine Aufgabe der vorliegenden Erfindung, ein Verfahren zur Erzeugung von Magnetresonanzaufnahmen und eine geeignete Steuervorrichtung zur Durchführung des Verfahrens bereitzustellen, mit dem das Sichtfeld eines Magnetresonanztomographen kostengünstig erweitert werden kann.

Diese Aufgabe wird durch ein Verfahren gemäß Patentanspruch 1 sowie durch ein Magnetresonanztomographiesystem gemäß Patentanspruch 10 gelöst.

Das eingangs genannte Verfahren zur Erzeugung von Magnetresonanzaufnahmen eines Untersuchungsobjekts in einem Magnetresonanztomographiegerät weist zumindest die folgenden Schritte auf:
Zunächst werden erste Magnetresonanzrohdaten in einem ersten Aufnahmebereich innerhalb eines Homogenitätsvolumens eines Grundmagnetfelds des Magnetresonanztomographiegeräts in einem k-Raum akquiriert.

Zusätzlich werden zweite Magnetresonanzdaten in einem zweiten Aufnahmebereich außerhalb des Homogenitätsvolumens des Magnetresonanztomographiegeräts unter Verwendung einer Signal-Kodierungsfunktion, welche nicht in Form eines herkömmlichen Fourier-Integrals vorliegt, und daher nicht in einem k-Raum akquiriert.

Der erste und zweite Aufnahmebereich stellen dabei räumliche Bereiche dar, in denen sich die durch die später rekonstruierte Bilddaten abgebildeten Bereiche befinden, unabhängig davon, ob es sich bei diesen abgebildete Bereichen um ein dreidimensionales Volumen oder eine Schicht handelt.

Die Akquirierung der ersten Magnetresonanzrohdaten und der zweiten Magnetresonanzdaten kann sowohl zeitgleich erfolgen, als auch hintereinander.

Auf Basis der ersten Magnetresonanzrohdaten werden erste Bilddaten mittels einer Fouriertransformation rekonstruiert und auf Basis der zweiten Magnetresonanzrohdaten werden zweite Bilddaten durch ein signal-modellbasiertes Bildrekonstruktionsverfahren rekonstruiert. Bei den Bildpunkten der Bilddaten kann es sich, je nachdem, ob Schichtbilddaten oder Volumenbilddaten rekonstruiert wurden, um Pixel oder Voxel handeln.

Zumindest vor der Erfassung der zweiten Magnetresonanzrohdaten werden eine magnetische Feldverteilung und eine magnetische Feldverteilung der Gradienten-Nichtlinearität zumindest im zweiten Aufnahmebereich ermittelt.

Die Reihenfolge, wann welche Bilddaten rekonstruiert werden, kann dabei zunächst beliebig sein. Wurden beispielsweise die ersten Magnetresonanzrohdaten vor den zweiten Magnetresonanzrohdaten aufgenommen, so kann beispielsweise auch die Rekonstruktion der ersten Bilddaten erfolgen, während die zweiten Magnetresonanzrohdaten erst akquiriert werden. Ebenso können auch die zweiten Magnetresonanzrohdaten vor den ersten Magnetresonanzrohdaten akquiriert werden und z.B. die zweiten Bilddaten zuerst rekonstruiert werden. Aber auch eine zeitgleiche Rekonstruktion der ersten Bilddaten und der zweiten Bilddaten ist möglich.

Die ersten Bilddaten und die zweiten Bilddaten werden erfindungsgemäß zu Kombinations-Bilddaten kombiniert, welche einen Bereich abdecken, der sich in dem ersten Aufnahmebereich und dem zweiten Aufnahmebereich erstreckt. Die Kombinations-Bilddaten können dabei durchgehenden Schnittbildern oder Volumen mit einem erweiterten Sichtfeld entsprechen, welche weit über das Homogenitätsvolumen hinausreichen.

Diese Erweiterung des Sichtfelds kann im einfachsten Fall mittels einer softwareseitigen Ein- bzw. Umstellung ermöglicht werden. Es bedarf somit also keinem erhöhten technischen Mehraufwand, wie dies beispielsweise durch Einbringen von zusätzlichen supraleitenden Spulen der Fall ist. Dadurch treten auch keine extremen Kostensteigerungen für die Herstellung eines Magnetresonanztomographiesystems auf, um das Sichtfeld zu vergrößern.

Ein entsprechendes erfindungsgemäßes Magnetresonanztomographiesystem ist zur Durchführung eines oben genannten erfindungsgemäßen Verfahrens ausgestaltet, und umfasst ein Magnetresonanztomographiegerät und eine Steuereinrichtung zur Steuerung des Magnetresonanztomographiegeräts, wobei die Steuereinrichtung zumindest die folgenden Komponenten umfasst:
- eine Akquisitionssteuereinheit, die dazu ausgelegt ist, das Magnetresonanzgerät zu veranlassen, Magnetresonanzdaten zu akquirieren, wobei erste

Magnetresonanzrohdaten in einem ersten Aufnahmebereich innerhalb eines Homogenitätsvolumens des Magnetresonanztomographiegeräts in einem k-Raum akquiriert werden und zweite Magnetresonanzrohdaten in einem zweiten Aufnahmebereich außerhalb des Homogenitätsvolumens des Magnetresonanztomographiegeräts unter Verwendung einer Signal-Kodierungsfunktion, welche nicht in Form eines herkömmlichen Fourier-Integrals vorliegt, und daher nicht in einem k-Raum akquiriert werden,
- eine Bildrekonstruktionseinheit, die dazu ausgelegt ist, erste Bilddaten, auf Basis der ersten Magnetresonanzrohdaten mittels einer Fouriertransformation und zweite Bilddaten auf Basis der zweiten Magnetresonanzrohdaten durch ein signal-modellbasiertes Bildrekonstruktionsverfahren zu rekonstruieren,
- eine Kombinationseinheit, die dazu ausgelegt ist, die ersten Bilddaten und die zweiten Bilddaten zu den bereits oben genannten Kombinationsbilddaten zu kombinieren, welche einen Bereich abdecken, der sich in dem ersten Aufnahmebereich und in dem zweiten Aufnahmebereich erstreckt.

Soll ein bereits bestehendes Magnetresonanztomographiesystem ein erweitertes Sichtfeld (im Sinne der vorangegangenen Beschreibung) aufweisen, so kann das Magnetresonanztomographiesystem wie nachfolgend beschrieben nachgerüstet werden. Ein Magnetresonanztomographiesystem kann aber auch direkt während der Fertigung mit den oben genannten Merkmalen ausgestaltet werden, um so ein erweitertes Sichtfeld zu erzielen.

Ein Großteil der zuvor genannten Komponenten der Steuereinrichtung, insbesondere die Akquisitionssteuereinheit, die Bildrekonstruktionseinheit und die Kombinationseinheit, können ganz oder teilweise in Form von Softwaremodulen in einem Prozessor einer entsprechenden Steuereinrichtung realisiert werden. Eine weitgehend softwaremäßige Realisierung hat den Vorteil, dass auch schon bisher verwendete Steuereinrichtungen auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung einer Steuereinrichtung eines Magnetresonanztomographiesystems ladbar ist, mit Programmabschnitten, um alle Schritte des erfindungsgemäßen Verfahrens auszuführen, wenn das Programm in der Steuereinrichtung des Magnetresonanztomographiesystems ausgeführt wird. Ein solches Computerprogrammprodukt kann neben dem Computerprogramm gegebenenfalls zusätzliche Bestandteile wie z. B. eine Dokumentation und/oder zusätzliche Komponenten auch Hardware-Komponenten, wie z.B. Hardware-Schlüssel (Dongles etc.) zur Nutzung der Software, umfassen.

Zum Transport zur Steuereinrichtung und/oder zur Speicherung an oder in der Steuereinrichtung kann ein computerlesbares Medium, beispielsweise ein Memorystick, eine Festplatte oder ein sonstiger transportabler oder fest eingebauter Datenträger dienen, auf welchem die von einer Rechnereinheit der Steuereinrichtung einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Weitere, besonders vorteilhafte Ausgestaltungen und Weiterbildungen der Erfindung ergeben sich aus den abhängigen Ansprüchen sowie der nachfolgenden Beschreibung, wobei die Ansprüche einer Anspruchskategorie auch analog zu den Ansprüchen und Beschreibungsteilen zu einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Die innerhalb eines Homogenitätsvolumens akquirierten ersten Magnetresonanzrohdaten werden in einem k-Raum akquiriert, um daraufhin bzw. daraus die ersten Bilddaten mittels einer Fourier-Transformation bzw. einer Fast-Fourier-Transformation FFT zu rekonstruieren. Hierbei handelt es sich um eine schnelle Rekonstruktionsmethode, für die nahezu alle bestehenden Magnetresonanztomographiesysteme ohnehin eingerichtet sind.

Die Feldinhomogenitäten im zweiten Aufnahmebereich außerhalb des Homogenitätsvolumens des Magnetresonanztomographiegeräts, in welchem die zweiten Magnetresonanzrohdaten akquiriert werden, unterliegen jedoch meist nicht Fourier physikalischen Effekten. Dies bedeutet, dass die Signal-Kodierungsfunktion, die hier verwendet wird, nicht in der Form eines herkömmlichen Fourier-Integrals eines Magnetresonanztomographiesystems vorliegt. Daher können auch die erfassten Magnetresonanzrohdaten nicht wie im Stand der Technik meist üblich in einem k-Raum akquiriert bzw. angeordnet werden. Folglich kann die Bildrekonstruktion nicht durch eine direkte k-Raum-Inversion, wie bei der kartesischen Magnetresonanztomographie mit linearen Gradienten und homogenen Magnetfeldern erreicht werden. Aus diesem Grund werden die zweiten Bilddaten auf Basis der zweiten Magnetresonanzrohdaten durch ein signal-modellbasiertes Bildrekonstruktionsverfahren rekonstruiert. Hierbei wird ein Signalmodell erzeugt, das die erwarteten Amplituden und Phasen gemessener Echosignale als eine Funktion des Kodierschemas zurückgibt. (Siehe hierzu z. B. Fessler IEEE Signal Processing Magazine [81] July (2010).)

Die Bildpunkte (also z. B. Pixel bzw. Voxel) der ersten Bilddaten, die auf den ersten Magnetresonanzrohdaten basieren und die innerhalb eines Homogenitätsvolumens akquiriert worden sind, weisen meist eine isotrope Gestalt auf. Die Bildpunkte der zweiten Bilddaten weisen jedoch meist eine anisotrope Gestalt auf. Dies liegt daran, dass die zweiten Bilddaten auf Basis der zweiten Magnetresonanzrohdaten außerhalb des Homogenitätsvolumens bzw. im Inhomogenitätsvolumen akquiriert worden sind. Im Inhomogenitätsvolumen ist jedoch das Magnetfeld verzerrt. Dies begründet sich dadurch, dass nicht in jeder Richtung (in x-, y-, z-Richtung) das Magnetfeld über derselben Strecke denselben Wert aufweist. Die Bildpunkte der zweiten Bilddaten haben also für definierte Ortskodierungs-Frequenzwerte und Ortskodierungs-Phasen unter Umständen auch unterschiedliche Ausdehnungen.

Werden nun die ersten Bilddaten und zweiten Bilddaten zu Kombinations-Bilddaten kombiniert, so werden bevorzugt in einem Schritt die ersten Bilddaten so rekonstruiert, dass sie auf einem ersten isotropen Gitter abgebildet sind, z.B. mit der bereits genannten Fourier-Transformation, vorzugsweise mit einer FFT.

Die Bildpunkte der zweiten Bilddaten werden dagegen auf Grund ihrer anisotropen Gestalt nach einer Rekonstruktion bevorzugt mittels eines Regridding-Verfahrens (bei der von einer Gitterauflösung zu einer anderen Gitterauflösung interpoliert wird) auf ein isotropes Gitter abgebildet, welches an das erste isotrope Gitter angepasst ist. Das heißt es wird bevorzugt dafür gesorgt, dass das isotrope Gitter, auf dem die Bildpunkte der zweiten Bilddaten abgebildet sind, also eine gleiche Gitterweite bzw. ein gleiches Gittermaß aufweist, wie das erste isotrope Gitter sowie eine angepasste Position der Bildpunkte unter Bildung einer Erweiterung des ersten isotropen Gitters nach außen.

Um grundsätzlich Bildpunkte aus einem anisotropen Gitter mit unterschiedlichen horizontalen und vertikalen Auflösungen in ein projiziertes und isotropes Gitter zu transformieren sind bereits dem Fachmann diverse Werkzeuge und Methoden bekannt. Beispiele hierfür sind die Software bzw. Softwarebibliotheken die im Rahmen der Projekte "ESMF Python Regridding Interface" (ESMPy) (siehe www.earthsystemcog.org/projects/esmpy/) oder "Modular Aerodynamic Design Computational Analysis Process (MADCAP)" (www.grc.nasa.gov/www/winddocs/madcap/ggs.html/) für den Bereich von Klima-, Wetter- und Winddaten angeboten werden, deren mathematischen Methoden aber hier genutzt werden können.

Erfindungsgemäß werden die ersten Magnetresonanzrohdaten innerhalb des Homogenitätsvolumens akquiriert. Die zweiten Magnetresonanzdaten werden hingegen außerhalb des Homogenitätsvolumens bzw. in einem Inhomogenitätsvolumen oder Inhomogenitätsbereich akquiriert. Dabei werden sie bei einer 2D-Magnetresonanztomographie(MR)-Sequenz, bevorzugt auf sogenannten Iso-Frequenzkonturen, die in etwa wellenförmigen bzw. sternförmigen Ringen entsprechen, akquiriert. Bei einer 3D-Magnetresonanztomographie(MR)-Sequenz werden die Magnetresonanzrohdaten bevorzugt auf Iso-Frequenzoberflächen, welche besonders bevorzugt ungefähre wellenförmige Oberflächen darstellen, eines relativ konstanten Grundmagnetfelds des Magnetresonanztomographiegeräts aufgenommen. Die Iso-Frequenzkonturen und Iso-Frequenzoberflächen stellen dabei Konturen bzw. Oberflächen dar, mit einer konstanten Grundmagnetfeldabweichung. Daher ist auch die Resonanzfrequenz entlang einer Iso-Frequenzkontur bzw. entlang einer Iso-Frequenzoberfläche gleich.

Eine 2D-MR-Sequenz und eine 3D-MR-Sequenz beschreiben dabei Messsequenzen, welche eine genaue zeitliche Abfolge von HF-Pulsen, der Änderung des Gradientenfeldes sowie der Erfassung von Messwerten festlegen.

Werden die ersten Magnetresonanzrohdaten mittels einer 2D-MR-Sequenz akquiriert, so umfassen die Magnetresonanzrohdaten Schicht-Rohdaten. Gleiches gilt für die zweiten Magnetresonanzrohdaten. Werden diese mittels einer 2D-MR-Sequenz akquiriert, so umfassen die zweiten Magnetresonanzrohdaten ebenfalls Schicht-Rohdaten.

Werden die ersten Magnetresonanzrohdaten hingegen mittels einer 3D-MR-Sequenz akquiriert, so umfassen die Magnetresonanzrohdaten Volumen-Rohdaten. Auch hier gilt das gleiche für die zweiten Magnetresonanzrohdaten. Werden diese mittels einer 3D-MR-Sequenz akquiriert, so umfassen die zweiten Magnetresonanzrohdaten Volumen-Rohdaten.

Es ist dabei nicht erforderlich, dass die Akquisition der ersten Magnetresonanzrohdaten und der zweiten Magnetresonanzrohdaten mit der gleichen MR-Sequenz erfolgt. So können beispielsweise die ersten Magnetresonanzrohdaten während einer 2D-MR-Sequenz und die zweiten Magnetresonanzrohdaten während einer 3D-MR-Sequenz akquiriert werden oder aber auch andersherum.

Zudem können auch die akquirierten Schicht-Rohdaten während einer Rekonstruktion zu einem Bildvolumen zusammengefasst werden und so eine 3D-Ansicht vom Inneren eines Untersuchungsobjekts wiedergeben.

Zur Akquisition der ersten und zweiten Magnetresonanzrohdaten können verschiedene Verfahren angewendet werden.

Bevorzugt kann z.B. ein MR-Spin-Echo-Signal zur Akquisition der Magnetresonanzrohdaten verwendet werden.

Ebenso bevorzugt, und daher nachfolgend exemplarisch beschrieben, wird aber zur Akquisition ein MR-Gradienten-Echo-Signal erzeugt, zur Echozeit TE mit einem Frequenzkodierungspuls. Bei dem Frequenzkodierungspuls kann es sich um den Gx-, Gy- oder Gz-Gradienten handeln, oder um eine Kombination dieser.

Zur Akquisition bzw. Erfassung der zweiten Magnetresonanzrohdaten, während einer 2D-MR-Sequenz, wird vorzugsweise ein schichtselektiver HF-Anregungspuls mit einer begrenzten Bandbreite, durch welche nur eine begrenzte Anzahl von Schichten angeregt wird, ausgespielt. Durch die begrenzte Bandbreite des HF-Anregungspuls wird zum einen eine Schichtbreite, z B. entlang der Z-Achse, kontrolliert und zum anderen die Ausdehnung von Pixeln entlang einer ausgewählten Frequenzkontur. Der HF-Anregungspuls weist dabei vorzugsweise eine mittlere Frequenz auf, die einem mittleren Magnetfeldwert entlang einer ausgewählten Frequenzkontur entspricht, z. B. in Resonanz zu einer mittleren Frequenz mit einer B₀-Magnetfeldabweichung + 10 ppm und mit einer Bandbreite von z. B. ± 2 ppm um die B₀-Magnetfeldabweichung + 10 ppm.

Bevorzugt wird dann passend, das heißt zeitlich korreliert, zum schichtselektiven HF-Anregungspuls ein bipolarer Schichtgradientenpuls, d.h. ein schichtselektiver Gradientenpuls, ausgespielt. Bevorzugt handelt es sich bei dem Schichtgradientenpuls um den Gz-Gradienten, um so ebenfalls die Schichtposition, beispielsweise entlang der Z-Achse, zu kontrollieren. Durch die Bipolarität des Schichtgradientenpulses geraten alle angeregten Spins in eine gleiche Phase, d. h. es kommt zu einer Refokussierung der angeregten Spins. Danach erfolgt in einem Auslesefenster die Auslese.

Wie erwähnt kann auch eine 3D-MR-Sequenz zur Erfassung von Volumen-Rohdaten genutzt werden. Bei einer 3D-MR-Sequenz zur Akquisition der zweiten Magnetresonanzrohdaten im zweiten Aufnahmebereich, wird vorzugsweise ein nichtschichtselektiver HF-Anregungspuls ausgespielt, wobei es zwar auch zu einer Einstrahlung des HF-Anregungspuls mit einer begrenzten Bandbreite kommt, aber ohne Schichtgradientenpuls. Der HF-Anregungspuls mit begrenzter Bandbreite weist auch hier vorzugsweise eine mittlere Frequenz auf, die einem mittleren Magnetfeldwert auf einer ausgewählten Frequenzoberfläche entspricht.

Vorzugsweise wird passend nachlaufend zum nichtschichtselektiven HF-Anregungspuls ein Phasenkodierungspuls ausgespielt. Der Phasenkodierungspuls kommt passend zeitlich korreliert zu einem Frequenzkodierungspuls an. Es kann sich bei dem Phasenkodierungspuls um den Gx-, Gy- oder Gz-Gradienten handeln oder auch um eine Kombination dieser. Danach erfolgt in einem Auslesefenster die Auslese.

Bei einer 3D-MR-Sequenz könnte prinzipiell aber auch ein schichtselektiver HF-Anregungspuls verwendet werden. Dabei wird beispielsweise eine relativ dicke Schicht von beispielsweise 15 cm aufgenommen. Diese Schicht enthält wiederum relativ dünne Schichten von beispielsweise einem 1 mm, die einen Abstand von beispielsweise 3 mm zueinander aufweisen. Dieser so entstehende "Schichtenkamm" wird solange verschoben, bis ein ganzer Aufnahmebereich abgedeckt ist. Im Inhomogenitätsbereich werden die Schichten jedoch nur entlang der Iso-Frequenzoberflächen verschoben. Die so entstandenen Schichten werden auch als "simultanious Multislices" beschrieben.

Die oben beschriebenen 2D-MR-Sequenzen oder 3D-MR-Sequenzen können vorzugsweise periodisch wiederholt werden, mit einer Repetitionszeit TR (Repetitionszeit TR = Zeit, bis aufeinanderfolgende Pulsfolgen auf dieselbe Schicht angewandt werden) für die verschiedenen möglichen orthogonalen Kombinationen von Phasen- und Frequenzkodiergradienten. Außerdem können die oben beschriebenen MR-Sequenzen iterativ wiederholt werden, mit der Repetitionszeit TR für unterschiedliche Iso-Frequenzkonturen, oder dementsprechende Iso-Frequenzoberflächen, bis ein "Ziel-Sichtfeld" bzw. eine Zielgröße für ein Sichtfeld erreicht ist.

Für die Rekonstruktion der Bilddaten, aber auch für die optimale Einstellung von Parametern für die verschiedenen MR-Sequenzen ist es hilfreich, im Vorhinein die inhomogene Feldverteilung zu kennen, als auch die Nichtlinearität der Gradienten außerhalb des Homogenitätsvolumens. Hierzu wird, zumindest vor einer Erfassung der zweiten Magnetresonanzrohdaten, vorzugsweise vor Aussendung des HF-Anregungspulses eine magnetische Feldverteilung zumindest im zweiten Aufnahmebereich außerhalb des Homogenitätsvolumens ermittelt. Die Ermittlung der magnetischen Feldverteilung kann vorzugsweise auch vor Ausspielung des Schichtgradientenpulses oder aber auch vor Ausspielung des Phasenkodierungspulses erfolgen. Die magnetische Feldverteilung kann dazu beispielsweise während einer Vorkalibrierung gemessen, berechnet oder aber auch simuliert werden. Z. B. kann eine Art Karte der Magnetfeldverteilung beim Scannen eines Patienten (also der Akquirierung der Magnetresonanzrohdaten) erzeugt werden, welche beispielsweise aus den Kalibrierungsdaten ausgelesen werden kann.

Zur Messung der magnetischen Feldverteilung können beispielsweise geeignete dynamische Magnetfeldkameras benutzt werden. Aber auch Temperatursensoren können eingesetzt werden, um beispielsweise eine Magnetfeldverschiebungen aufgrund von thermischen Effekten zu berücksichtigen. Dafür könnten beispielsweise mehrere Kalibrierdatensätze gemessen und gespeichert werden, für einen bestimmten Spannbereich von Betriebstemperaturen des Magnetresonanztomographiesystems.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:
Figur 1 eine schematische Darstellung eines Magnetresonanztomographiesystems gemäß einem Ausführungsbeispiel der Erfindung,
Figur 2 eine schematische Darstellung des sphärischen Bildvolumens SIV des statischen B₀-Magnetfelds (B₀-Homogenitätsvolumen SHV) und eines B₀-Inhomogenitätsvolumens SIHV außerhalb des sphärischen Bildvolumens SIV eines Magnetresonanztomographiesystems,
Figur 3 einen Schnitt durch das B₀-Homogenitätsvolumen SHV und das B₀-Inhomogenitätsvolumen SIHV gemäß Figur 2 in einer transversalen Schichtebene,
Figur 4 einen Schnitt durch das B₀-Homogenitätsvolumen SHV und das B₀-Inhomogenitätsvolumen SIHV gemäß Figur 2 in einer sagittalen Schichtebene,
Figur 5 einen Schnitt durch das B₀-Homogenitätsvolumen SHV und das B₀-Inhomogenitätsvolumen SIHV gemäß Figur 2 in einer koronalen Schichtebene,
Figur 6 ein Blockdiagramm für einen möglichen Ablauf des erfindungsgemäßen Verfahrens,
Figur 7 eine beispielhafte Pulssequenz, die zur Durchführung des erfindungsgemäßen Verfahrens verwendet werden kann,
Figur 8 eine weitere beispielhafte Pulssequenz, die zur Durchführung des erfindungsgemäßen Verfahrens verwendet werden kann,
Figur 9 eine schematische Darstellung einer mittels des erfindungsgemäßen Verfahrens zur Erzeugung von Schichtbilddaten erfassbaren Schicht, welche sich durchgehend innerhalb eines Homogenitätsvolumens und außerhalb eines Homogenitätsvolumens des B₀-Magnetfelds erstreckt,
Figur 10 eine schematische Darstellung einer mittels des erfindungsgemäßen Verfahrens zur Erzeugung von Volumenbilddaten erfassbaren räumlichen Volumenbereichs, welcher sich durchgehend innerhalb eines Homogenitätsvolumens und außerhalb eines Homogenitätsvolumens des B₀-Magnetfelds erstreckt,
Figur 11 eine schematische Darstellung eines isotropen Gitters,
Figur 12 eine schematische Darstellung eines anisotropen Gitters.

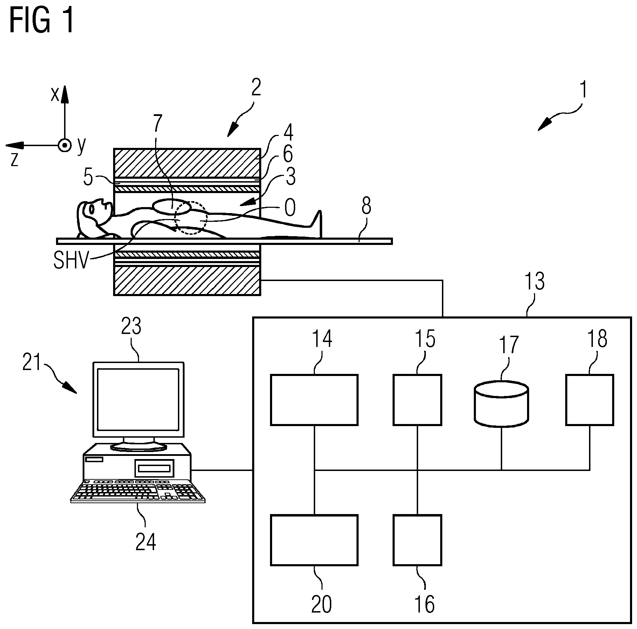

In Figur 1 ist grob schematisch ein Magnetresonanztomographiesystem 1 dargestellt. Es umfasst zum einen das eigentliche Magnetresonanztomographiegerät 2 bzw. den Magnetresonanzscanner 2 mit einem Untersuchungsraum 3 bzw. Patiententunnel, in den auf einer Liege 8 ein Patient oder Proband positioniert ist, in dessen Körper sich das eigentliche Untersuchungsobjekt O befindet, bzw. bei dem ein bestimmter Körperbereich das Untersuchungsobjekt O darstellt.

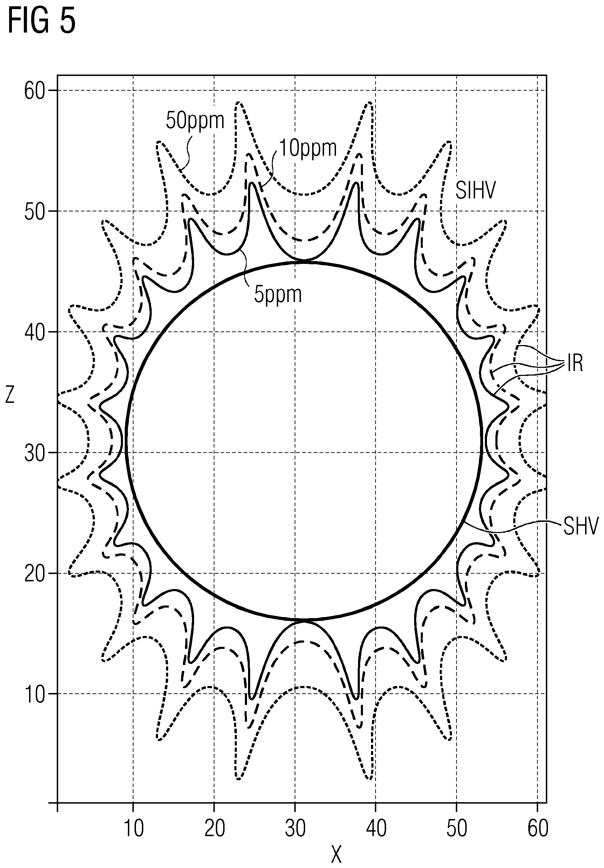

Der Magnetresonanzscanner 2 ist in üblicher Weise mit einem Grundfeldmagnetsystem 4, einem Gradientensystem 6 sowie einem HF-Sendeantennensystem 5 und einem HF-Empfangsantennen-System 7 ausgestattet. In dem dargestellten Ausführungsbeispiel handelt es sich bei dem HF-Sendeantennensystem 5 um eine im Magnetresonanzscanner 2 fest eingebaute Ganzkörperspule, wogegen das HF-Empfangsantennensystem 7 aus am Patienten bzw. Probanden anzuordnenden Lokalspulen besteht (in Figur 5 nur durch eine einzelne Lokalspule symbolisiert). Grundsätzlich können aber auch die Ganzkörperspule als HF-Empfangsantennensystem und die Lokalspulen als HF-Sendeantennensystem genutzt werden, sofern diese Spulen jeweils in unterschiedliche Betriebsweisen umschaltbar sind. Das Gradientensystem 6 umfasst in üblicher Weise einzeln ansteuerbare Gradientenspulen, um unabhängig voneinander Gradienten in x-, y- oder z-Richtung schalten zu können. Zudem enthält der Magnetresonanzscanner 2 (nicht dargestellte) Shimspulen, die in üblicher Weise ausgebildet sein können.

Das Grundfeldmagnetsystem 4 besteht aus mehreren Magnetspulen und ist hier in üblicher Weise so ausgebildet, dass es ein Grundmagnetfeld B₀ in Längsrichtung des Patienten erzeugt, d. h. entlang der in z-Richtung verlaufenden Längsachse des Magnetresonanzscanners 2 sowie einen Homogenitätsbereich des Grundmagnetfelds B₀, in welchem das Grundmagnetfeld B₀ homogen ist. Der Homogenitätsbereich entspricht hier einem kugelförmigen Homogenitätsvolumen SHV, welcher in gestrichelter Linie eingezeichnet ist und innerhalb des Patiententunnels 3 liegt. Im Ursprung des Homogenitätsvolumens SHV befindet sich das Iso-Zentrum des Magnetresonanzscanners 2.

Das Magnetresonanztomographiesystem 1 weist weiterhin eine zentrale Steuereinheit 13 auf, die zur Steuerung des gesamten Magnetresonanztomographiesystems 1 verwendet wird. Die zentrale Steuereinheit 13 umfasst eine Akquisitionssteuereinheit bzw. Bildaufnahmeeinheit 14 zur Pulssequenzsteuerung. In dieser werden die Abfolgen von HF-Pulsen und von Gradientenpulsen in Abhängigkeit von einer gewählten Bildgebungssequenz gesteuert. Zur Ausgabe der einzelnen HF-Pulse weist die zentrale Steuereinheit 13 eine HF-Einheit 15 und zur Steuerung der Gradientenspulen eine Gradienteneinheit 16 auf, welche mit der Bildaufnahmeeinheit 14 zur Aussendung der Pulssequenzen entsprechend kommunizieren. Die HF-Einheit 15 umfasst dabei nicht nur ein Sendeteil, um die HF-Pulssequenzen auszusenden, sondern auch ein Empfangsteil, um koordiniert Magnetresonanzrohdaten zu akquirieren.

Eine Rekonstruktionseinheit 20 übernimmt die akquirierten Rohdaten und rekonstruiert daraus die Bilddaten. Diese Bilddaten können dann beispielsweise in einem Speicher 17 hinterlegt werden.

Wie durch ein Einstrahlen von HF-Pulsen und die Erzeugung von Gradientenfeldern geeignete Rohdaten akquiriert und daraus MR-Bilder rekonstruiert werden können, ist dem Fachmann grundsätzlich bekannt und wird hier nicht näher erläutert.

Die rekonstruierten Bilddaten können dann - wie später noch erläutert wird - in einer Kombinationseinheit 18 zu Kombinations-Bilddaten BDK kombiniert werden, um ein finales homogenes MR-Bild zu erhalten.

Eine Bedienung der zentralen Steuereinheit 13 kann über eine Terminalschnittstelle 21 mittels einer Eingabeeinheit 24 und einer Anzeigeeinheit 23 erfolgen, über die somit auch das gesamte Magnetresonanztomographiesystem 1 durch eine Bedienperson bedient werden kann. Auf der Anzeigeeinheit 23 können auch MR-Bilder angezeigt werden, und mittels der Eingabeeinheit 24 ggf. in Kombination mit der Anzeigeeinheit 23 können Messungen geplant und gestartet werden.

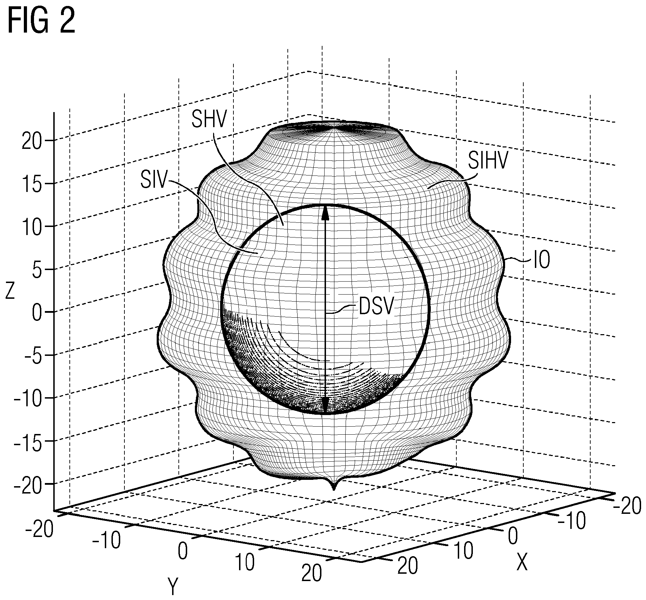

Figur 2 zeigt beispielhaft eine Magnetfeldverteilung mit dem Homogenitätsvolumen SHV aus Figur 1. In diesem Homogenitätsvolumen SHV ist eine Schwankung des herrschenden Grundmagnetfelds B₀ kleiner als 20 ppm peak-to-peak oder weniger als 3 ppm RMS. Auf dieses Messvolumen, dem sphärische Bildvolumen SIV mit einem Durchmesser DSV, welches die oben genannten Toleranzangaben einhält, ist bisher die Akquisition von Magnetresonanzrohdaten RD1, RD2 begrenzt. Das heißt, dass ein Sichtfeld dem sphärischen Bildvolumen SIV gleichkommt. Außerhalb des Homogenitätsvolumens SHV befinden sich ein Inhomogenitätsvolumen SIHV. Die Oberfläche bzw. die Iso-Frequenzoberfläche der Magnetfeldabweichung ist hier sehr stark und komplex gewellt bzw. geschlängelt, wobei die Homogenität des statischen Magnetfelds B₀ eine größere Abweichung als z. B. 20 ppm peak-to-peak oder größer als 3 ppm RMS aufweist.

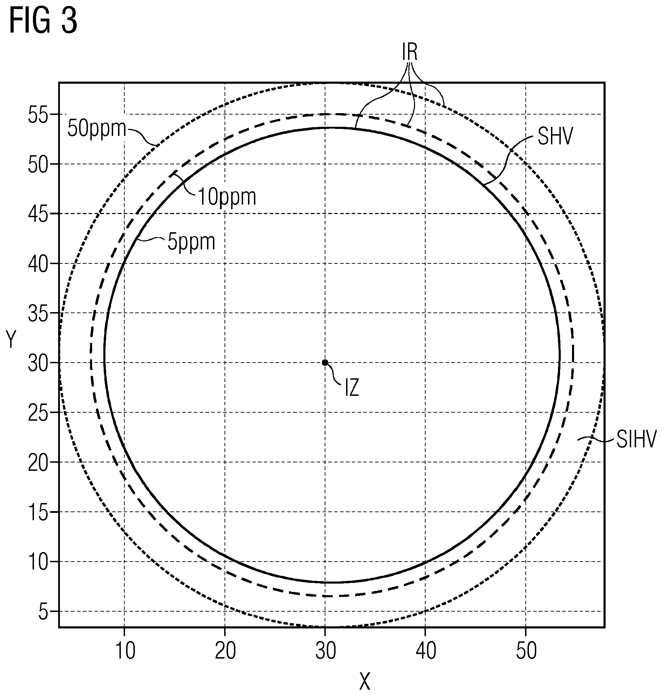

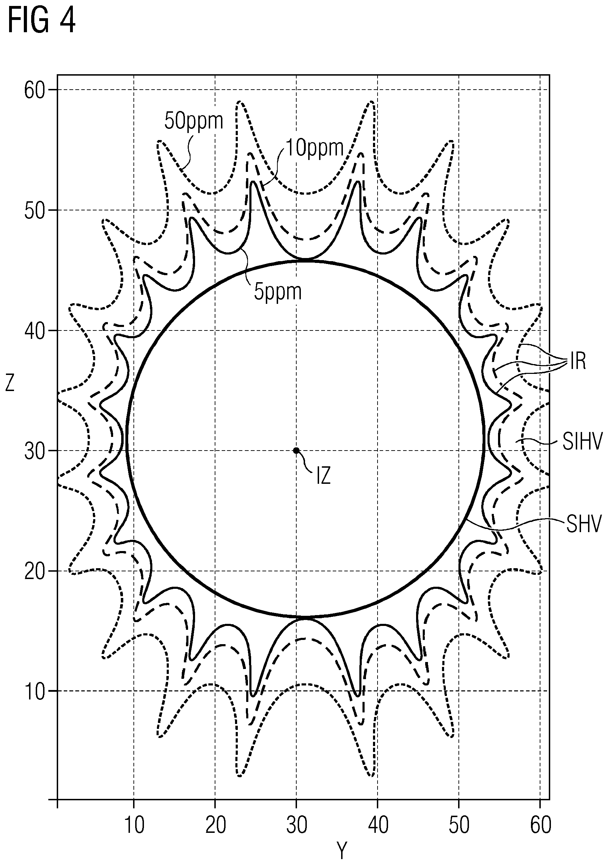

In den Figuren 3 bis 5 sind verschiedene Schnitte durch die Magnetfeldverteilung aus Figur 1 dargestellt. In Figur 3 ist die Magnetfeldabweichung in einer transversalen Schicht, d.h. in der x-y-Ebene senkrecht zur z-Achse(siehe Figur 1), in Figur 4 ist die Magnetfeldabweichung in einer sagittalen Schicht, d.h. in der y-z-Ebene senkrecht zur x-Achse, und in Figur 5 ist eine Magnetfeldabweichung in einer koronalen Schicht, d.h. in der x-z-Ebene senkrecht zur y-Achse, dargestellt.

In dem in Figur 3 gezeigten transversalen Schnitt durch das Grundmagnetfeld B₀ sind drei Linien IR gezeigt, die drei Iso-Frequenzkonturen IR darstellen, auf denen die Magnetfeldabweichung jeweils konstant verlaufen. In der Figur sind die Iso-Frequenzkonturen IR für eine Abweichung um 5 ppm, um 10 ppm und um 50 ppm gezeigt. Wie in Figur 3 zu sehen ist, steigt die Abweichung des Magnetfelds B₀ mit größer werdender Entfernung vom Iso-Zentrum IZ, d.h. je näher es an einen Randbereich des Ringtunnels reicht, erheblich an.

Wie aus den in den Figuren 4 und 5 ebenfalls gezeigten Iso-Frequenzkonturen IR für die Abweichungen um 5 ppm, um 10 ppm und um 50 ppm ersichtlich ist, erhöht und erniedrigt sich die Magnetfeldstärke des magnetischen Felds B₀ am Rand des Sichtfelds des Magnetresonanztomographen 2 in den anderen Ebenen bzw. Schnitten räumlich periodisch. Der Grund dafür ist die übliche Anordnung der Magnetfeldspulen für das Grundmagnetfeld B₀ im Magnetresonanzscanner 2. In dem in den Figuren 4 und 5 gezeigten Fall führen fünf Magnetfeldspulen zu entsprechenden Magnetfelderhöhungen (in der koronalen Darstellung jeweils auf der linken/rechten Seite) nahe diesen Spulen und zu entsprechenden Magnetfelderniedrigungen zwischen den Magnetfeldspulen. Dadurch ergibt sich das gezeigte periodische Magnetfeldverhalten.

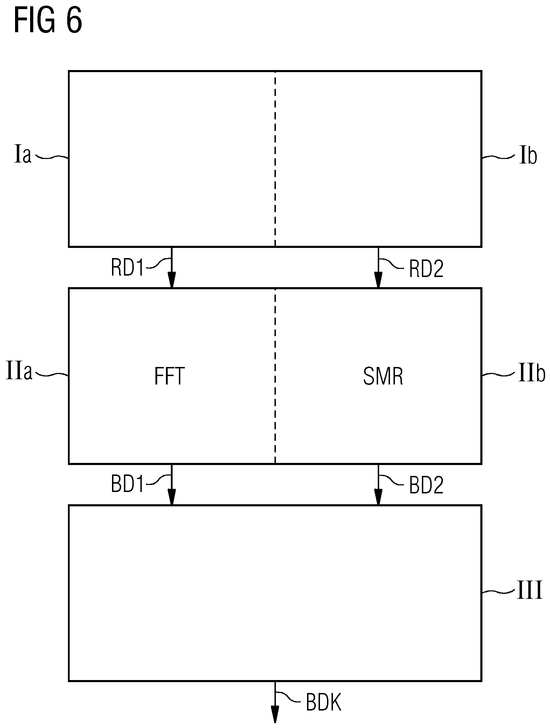

In Figur 6 ist ein Blockdiagramm gezeigt, mit dem ein Verfahren gemäß einem Ausführungsbeispiel der Erfindung veranschaulicht wird.

In einem ersten Verfahrensschritt Ia werden erste Magnetresonanzrohdaten RD1 in einem ersten Aufnahmebereich B1_{2D}, B1_{3D} innerhalb eines Homogenitätsvolumens SHV akquiriert.

Zeitgleich oder zeitlich versetzt (vorher oder nachher) zur

Akquisition der ersten Magnetresonanzrohdaten RD1 werden in einem Verfahrensschritt Ib zweite Magnetresonanzrohdaten RD2 in einem zweiten Aufnahmebereich B2_{2D}, B2_{3D} außerhalb des Homogenitätsvolumens SHV akquiriert.

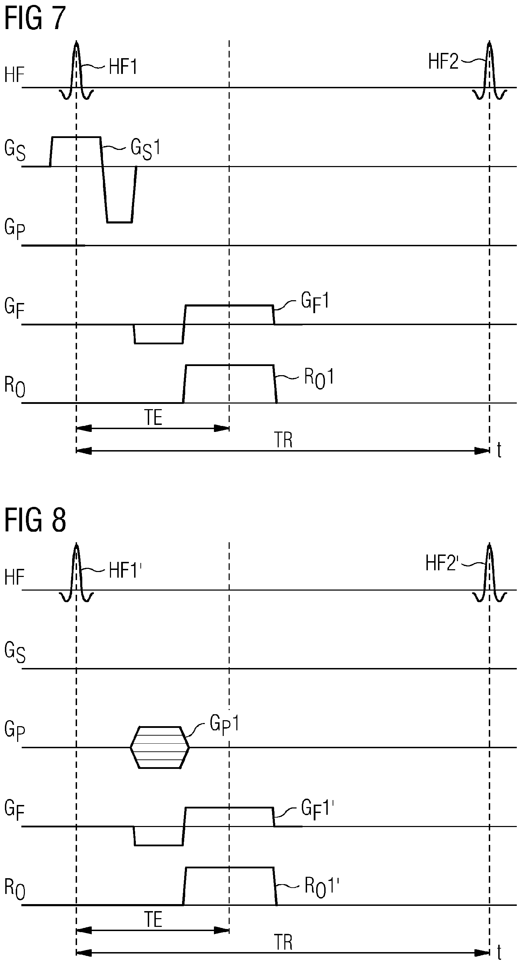

Figur 7 zeigt zur Akquisition der Magnetresonanzrohdaten in einem zweiten Aufnahmebereich B2_{2D} ein Pulsdiagramm, d.h. den zeitlichen Verlauf einer Pulssequenz, für eine 2D-MR-Sequenz gemäß einem Ausführungsbeispiel der Erfindung. In der ersten Zeile HF sind ein erster HF-Anregungspuls HF1 und ein zweiter HF-Anregungspuls HF2 gezeigt. In der zweiten Zeile G_{S} ist ein bipolarer Schichtgradientenpuls G_{S}1, d. h. ein schichtselektivem Gradientenpuls, gezeigt. Die Frequenzoffsets des HF-Anregungspulses HF1 bestimmen zusammen mit der Amplitude des bipolaren Schichtgradientenpulses G_{S}1 die Position und bzw. die Breite der angeregten Schichten. Die Frequenzoffsets bestimmen dabei eine angeregte Iso-Frequenzkontur IR. Der Schichtgradientenpuls G_{S}1 bestimmt dabei die angeregten Schichten entlang dieser Iso-Frequenzkontur IR, bzw. Iso-Kontur IR. Durch die Bipolarität des Schichtgradientenpulses G_{S}1 kommt es zu einer Refokussierung der angelegten Spins. In der vierten Zeile G_{F} wird ein Frequenzkodierungsgradient G_{F}1 nach dem bipolaren Schichtgradientenpuls G_{S}1 ausgespielt. Mit dem ebenfalls bipolaren Frequenzkodierungsgradienten G_{F}1 wird das Momentum zum Zeitpunkt TE ausgeglichen bzw. auf 0 gesetzt. In der fünften Zeile R_{O} ist ein Auslesefenster R_{O}1 dargestellt. Das Auslesen bzw. Empfangen der Magentresonanzssignale (die eigentliche Akquisition der Rohdaten) erfolgt zeitlich symmetrisch um diese 0-Linie des Momentums, da dann alle Spins umfasst sind. Nach der Repetitionszeit TR kann dann wieder ein HF-Anregungspuls HF2 ausgespielt werden, um die Sequenz iterativ zu wiederholen. Ein Phasenkodierungsgradient ist hierbei nicht nötig, da mehrere parallele Empfangsspulen verwendet werden.

Mit Hilfe des Frequenzkodierungsgradienten G_{F}1 erfolgt eine Abtastung des k-Raums, für die mit dem bipolaren Schichtgradientenpuls G_{S}1 ausgewählten Schichten. Die Trennung der akquirierten Rohdaten bzw. rekonstruierten Bilddaten der unterschiedlichen Schichten werden anschließend in einem Auswertungsschritt auf Basis der räumlichen Sensibilitätsprofile der Empfangsspulen, z. B. mit dem grundsätzlich bekannten Grappa-Verfahren oder dem SENSE-Verfahren, durchgeführt.

Die Akquirierung der ersten Magnetresonanzrohdaten RD1 und der zweiten Magnetresonanzrohdaten RD2 kann aber auch mittels einer 3D-MR-Sequenz erfolgen.

Figur 8 zeigt hierzu analog zu Figur 7 ein Pulsdiagramm für eine 3D-MR-Sequenz in einem zweiten Aufnahmebereich B2_{3D}. In der ersten Zeile HF ist ein HF-Anregungspuls HF1' gezeigt, der während einer Anregungsphase ausgespielt wird. Der HF-Anregungspuls HF1' weist auch hier eine begrenzte Bandbreite auf. Die HF-Anregungspulse bzw. Frequenzoffsets bestimmen somit die Iso-Frequenzoberflächen sowie durch die begrenzte Bandbreite auch die Dicke der angeregten Iso-Frequenzoberfläche IO. Ein Schichtgradientenpuls wird hier nicht benötigt. In der dritten Zeile G_{P} ist ein Phasenkodiergradient G_{P}1 dargestellt. Dies kann der Gx-, Gy- oder Gz-Gradient sein oder eine Kombination aus diesen Gradienten. In der vierten Zeile G_{F} ist ein Frequenzkodierungspuls G_{F}1' gezeigt, welcher genutzt wird, um ein MR-Gradientenechosignal zur Zeit TE zu erzeugen. Der Frequenzkodierungsgradient G_{F}1' kann der Gx-, Gy-, Gz-Gradient oder eine Kombination aus diesen Gradienten sein. Auch hier wird ein Auslesefenster R₀1', gezeigt in der fünften Zeile R₀, wieder um die 0-Linie des Momentums, da dann wie bereits erwähnt alle Spins in Phase sind.

In weiteren Verfahrensschritten IIa, IIb (siehe wieder Figur 6) werden dann die Bilddaten auf Basis der akquirierten Magnetresonanzrohdaten rekonstruiert.

Das Rekonstruktionsverfahren unterscheidet sich hier jedoch, für Bilddaten, die auf Basis von Magnetresonanzrohdaten rekonstruiert werden sollen, welche innerhalb eines Homogenitätsvolumens SHV erfasst wurden, und für Bilddaten, die auf Basis von Magnetresonanzrohdaten rekonstruiert werden sollen, die innerhalb eines Inhomogenitätsvolumen SIHV akquiriert worden sind.

Wurden die ersten Magnetresonanzrohdaten RD1 im Homogenitätsvolumen SHV akquiriert, so wurden sie - wie üblich - in einem k-Raum akquiriert. Daher können die ersten Bilddaten BD1 auf Basis der ersten Magnetresonanzrohdaten RD1 hier im Verfahrensschritt IIa mittels einer Fast-Fourier-Transformation FFT rekonstruiert werden.

Die zweiten Magnetresonanzrohdaten RD2, die im Inhomogenitätsvolumen SIHV akquiriert worden sind, werden nicht im k-Raum abgelegt bzw. wurden dort nicht akquiriert. In einem Verfahrensschritt IIb werden daher die zweiten Bilddaten BD2 mittels eines signal-modelbasierten Bildrekonstruktionsverfahren SMR auf Basis der zweiten Magnetresonanzrohdaten RD2 rekonstruiert. Dabei wird ein Signalmodell erzeugt, das die erwarteten Amplituden und Phasen gemessener Echosignale als eine Funktion des Kodierschemas zurückgibt.

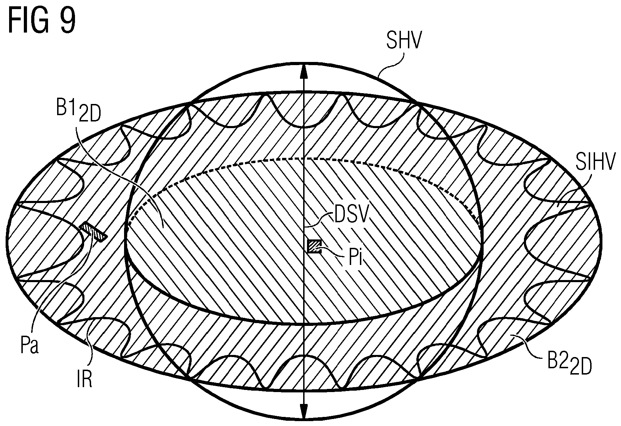

Figur 9 zeigt dazu beispielhaft Bildpunkte die als Schichtbilddaten rekonstruiert worden sind. Hierbei ist ein erster Aufnahmebereich B1_{2D}, in dem sich innerhalb des Homogenitätsvolumens SHV rekonstruierte Bilddaten befinden, und ein zweiter Aufnahmebereich B2_{2D}, in dem sich außerhalb des Homogenitätsvolumens SHV rekonstruierte Bilddaten befinden, zu sehen. Liegen die rekonstruierten Bilddaten innerhalb des Homogenitätsvolumens SHV handelt es sich um isotrope Pixel Pi. Bei den rekonstruierten Bilddaten im zweiten Aufnahmebereich B2_{2D} außerhalb des Homogenitätsvolumens SHV handelt es sich aber um anisotrope Pixel Pa. Dies liegt daran, dass das Magnetfeld B₀ im inhomogenen Bereich bzw. Inhomogenitätsbereich verzerrt ist. Das Magnetfeld B₀ weist dabei in unterschiedlichen Richtungen (x-, y- und z-Richtung) unterschiedliche Werte auf. Im zweiten Aufnahmebereich B2_{2D}, außerhalb des Homogenitätsvolumens SHV, können die anisotropen Pixel Pa also nicht, wie bereits erklärt, in einem k-Raum abgelegt und beispielsweise mit einer Fast-Fourier-Transformation FFT rekonstruiert werden.

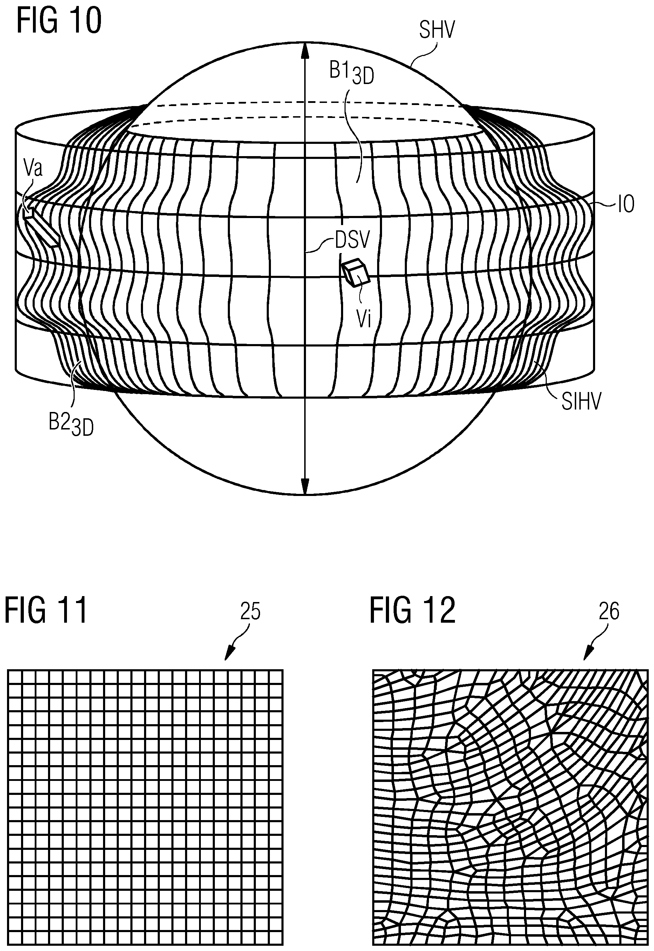

Wie in Figur 10 im Vergleich zu Figur 9 gezeigt, verhält es sich ähnlich, wenn die Bildpunkte als Volumenbilddaten rekonstruiert worden sind. Bei den Bildpunkten, die innerhalb des ersten Aufnahmebereichs B1_{3D} im Homogenitätsvolumens SHV rekonstruiert worden sind, handelt es sich um isotrope Voxel Vi. Auf Grund des unterschiedlich ausgedehnten Magnetfelds B₀ handelt es sich hier bei dem exemplarisch dargestellten Bildpunkt im zweiten Aufnahmebereich B2_{3D} außerhalb des Homogenitätsvolumens SHV um ein anisotropes Voxel Va. Auch hier ist im zweiten Aufnahmebereich B2_{3D} eine schnelle Fourier-Transformation nicht möglich.

Die in Figur 6 gezeigten Verfahrensschritte IIa, IIb zur Rekonstruktion der Bilddaten BD1, BD2 können zeitlich unabhängig voneinander, d.h. beliebig nacheinander oder zeitlich parallel bzw. auch geringfügig zeitlich versetzt, erfolgen.

In einem nachfolgenden Verfahrensschritt III zur Erzeugung eines finalen homogenen Magnetresonanztomographiebildes, werden die im Verfahrensschritt IIa rekonstruierten ersten Bilddaten BD1 und die im Verfahrensschritt IIb rekonstruierten zweiten Bilddaten BD2 zu Kombinations-Bilddaten BDK zusammengefügt.

Figur 11 zeigt hierzu ein isotropes Gitter 25, auf dem die isotropen Pixel Pi, bzw. Voxel Vi aus Figur 9 bzw. Figur 10 abgebildet werden können. Figur 12 zeigt im Gegensatz dazu ein Beispiel für ein anisotropes Gitter 26. Um insgesamt ein homogenes Bild zu bekommen, werden der erste Aufnahmebereich B1_{2D}, B1_{3D} und der zweite Aufnahmebereich B2_{2D}, B2_{3D} mit Hilfe sogenannter Regridding-Verfahren auf einem isotropen Gitter zusammengeführt. Konkret können hier die Bilddaten des zweiten Aufnahmebereichs B2_{2D}, B2_{3D} mit Hilfe des Regridding-Verfahrens an die Bilddaten des ersten Aufnahmebereichs B1_{2D}, B1_{3D}, d.h. das dafür bereits existierende isotrope Gitter angepasst werden. Dies gelingt z. B. durch eine Korrektur der Magnetfeld Nichtlinearität und einer Anpassung der Pixel- bzw. Voxelgröße sowie der Ortskodierung.

Es wird abschließend noch einmal darauf hingewiesen, dass es sich bei den vorhergehend detailliert beschriebenen Verfahren sowie bei dem dargestellten Magnetresonanztomographiesystem lediglich um Ausführungsbeispiele handelt, welche vom Fachmann in verschiedenster Weise modifiziert werden können, ohne den Bereich der Erfindung zu verlassen, vorausgesetzt, der resultierende Gegenstand fällt in den Schutzbereich der Erfindung, wie er in den Ansprüchen definiert ist. Weiterhin schließt die Verwendung der unbestimmten Artikel "ein" bzw. "eine" nicht aus, dass die betreffenden Merkmale auch mehrfach vorhanden sein können. Ebenso schließen die Begriffe "Einheit" und "Modul" nicht aus, dass die betreffenden Komponenten aus mehreren zusammenwirkenden Teil-Komponenten bestehen, die gegebenenfalls auch räumlich verteilt sein können.

## Patentansprüche

1. Verfahren zur Erzeugung von Magnetresonanzaufnahmen eines Untersuchungsobjekts (O) in einem Magnetresonanztomographiegerät (2), aufweisend die folgenden Schritte:
- Akquirierung von Magnetresonanzrohdaten (RD1, RD2), wobei erste Magnetresonanzrohdaten (RD1) in einem ersten Aufnahmebereich (B1_{2D}, B1_{3D}) innerhalb eines Homogenitätsvolumens (SHV) des Magnetresonanztomographiegeräts (2) in einem k-Raum akquiriert werden, und zweite Magnetresonanzrohdaten (RD2) in einem zweiten Aufnahmebereich (B2_{2D}, B2_{3D}) außerhalb des Homogenitätsvolumens (SHV) des Magnetresonanztomographiegeräts (2) unter Verwendung einer Signal-Kodierungsfunktion, welche nicht in Form eines herkömmlichen Fourier-Integrals vorliegt, und daher nicht in einem k-Raum akquiriert werden,
- Rekonstruieren von ersten Bilddaten (BD1) auf Basis der ersten Magnetresonanzrohdaten (RD1) und Rekonstruieren von zweiten Bilddaten (BD2) auf Basis der zweiten Magnetresonanzrohdaten (RD2), wobei
- die ersten Bilddaten (BD1) basierend auf den ersten Magnetresonanzrohdaten (RD1) mittels einer Fouriertransformation (FFT) rekonstruiert werden und
- die zweiten Bilddaten (BD2) auf Basis der zweiten Magnetresonanzrohdaten (RD2) durch ein signal-modellbasiertes Bildrekonstruktionsverfahren (SMR) rekonstruiert werden
- Kombination der ersten Bilddaten (BD1) und der zweiten Bilddaten (BD2) zu Kombinations-Bilddaten (BDK), welche einen Bereich abdecken, der sich in dem ersten Aufnahmebereich (B1_{2D}, B1_{3D}) und dem zweiten Aufnahmebereich (B2_{2D}, B2_{3D}) erstreckt,
wobei zumindest vor einer Erfassung der zweiten Magnetresonanzrohdaten (RD2) eine magnetische Feldverteilung und eine magnetische Feldverteilung der Gradienten-Nichtlinearität zumindest im zweiten Aufnahmebereich (B2_{2D}, B2_{3D}) ermittelt wird.

2. Verfahren nach Anspruch 1, wobei Bildpunkte der ersten Bilddaten (BD1) so rekonstruiert werden, dass sie auf einem ersten isotropen Gitter (25) abgebildet sind, und Bildpunkte der zweiten Bilddaten (BD2) nach einer Rekonstruktion mittels eines Regridding-Verfahrens auf ein isotropes Gitter abgebildet werden, welches an das erste isotrope Gitter (25) angepasst ist.

3. Verfahren nach einem der vorstehenden Ansprüche, wobei die zweiten Magnetresonanzrohdaten (RD2) auf Iso-Frequenzkonturen (IR) und/oder Iso-Frequenzoberflächen (IO) eines Grundmagnetfelds des Magnetresonanztomographiegeräts (2) aufgenommen werden.

4. Verfahren nach einem der vorstehenden Ansprüche, wobei die ersten Magnetresonanzrohdaten (RD1) und/oder die zweiten Magnetresonanzrohdaten (RD2) Schicht-Rohdaten umfassen.

5. Verfahren nach einem der vorstehenden Ansprüche, wobei die ersten Magnetresonanzrohdaten (RD1) und/oder die zweiten Magnetresonanzrohdaten (RD2) Volumen-Rohdaten umfassen.

6. Verfahren nach einem der vorstehenden Ansprüche, wobei zur Akquirierung von Magnetresonanzrohdaten (RD1, RD2) ein MR-Gradienten-Echo-Signal und/oder ein MR-Spin-Echo-Signal erzeugt wird.

7. Verfahren nach einem der vorstehenden Ansprüche, wobei zur Erfassung der zweiten Magnetresonanzrohdaten (RD2) ein schichtselektiver HF-Anregungspuls (HF1, HF2) ausgespielt wird, wobei vorzugsweise passend zum schichtselektiven HF-Anregungspuls (HF1, HF2) ein Bi-polarer Gradientenpuls (G_{S}1) ausgespielt wird.

8. Verfahren nach einem der vorstehenden Ansprüche, wobei zur Erfassung der zweiten Magnetresonanzrohdaten (RD2) ein nichtschichtselektiver HF-Anregungspuls (HF1', HF2') ausgespielt wird, wobei vorzugsweise nachlaufend zum HF-Anregungspuls (HF1', HF2') ein Phasenkodierungspuls (G_{P}1) ausgespielt wird.

9. Verfahren nach einem der vorstehenden Ansprüche, wobei die Ermittlung der magnetischen Feldverteilung und der magnetischen Feldverteilung der Gradienten-Nichtlinearität vor Aussendung des HF-Anregungspulses (HF1, HF1', HF2, HF2`) und/oder vor Ausspielung des Gradientenpulses (G_{S}1) und/oder des Phasenkodierungspulses (G_{P}1) erfolgt.

10. Magnetresonanztomographiesystem (1) umfassend ein Magnetresonanztomographiegerät und eine Steuereinrichtung (13) zur Steuerung des Magnetresonanztomographiegeräts (2),
wobei die Steuereinrichtung (13) zumindest umfasst:
- eine Akquisitionssteuereinheit (14), die dazu ausgelegt ist, das Magnetresonanztomographiegerät zu veranlassen, Magnetresonanzrohdaten (RD1, RD2) zu akquirieren (2), wobei erste Magnetresonanzrohdaten (RD1) in einem ersten Aufnahmebereich (B1_{2D}, B1_{3D}) innerhalb eines Homogenitätsvolumens (SHV) des Magnetresonanztomographiegeräts (2) in einem k-Raum akquiriert werden, und zweite Magnetresonanzrohdaten (RD2) in einem zweiten Aufnahmebereich (B2_{2D}, B2_{3D}) außerhalb des Homogenitätsvolumens (SHV) des Magnetresonanztomographiegeräts (2) unter Verwendung einer Signal-Kodierungsfunktion, welche nicht in Form eines herkömmlichen Fourier-Integrals vorliegt, und daher nicht in einem k-Raum akquiriert werden,
- eine Bildrekonstruktionseinheit (20) die dazu ausgelegt ist, erste Bilddaten (BD1) auf Basis der ersten Magnetresonanzrohdaten (RD1) und zweite Bilddaten (BD2) auf Basis der zweiten Magnetresonanzrohdaten (RD2) zu rekonstruieren, wobei
- die ersten Bilddaten (BD1) basierend auf den ersten Magnetresonanzrohdaten (RD1) mittels einer Fouriertransformation (FFT) rekonstruiert werden und
- die zweiten Bilddaten (BD2) auf Basis der zweiten Magnetresonanzrohdaten (RD2) durch ein signal-modellbasiertes Bildrekonstruktionsverfahren (SMR) rekonstruiert werden
- eine Kombinationseinheit (18) die dazu ausgelegt ist, die ersten Bilddaten (BD1) und die zweiten Bilddaten (BD2) zu Kombinations-Bilddaten (BDK) zu kombinieren, welche einen Bereich abdecken, der sich in dem ersten Aufnahmebereich (B1_{2D}, B1_{3D}) und dem zweiten Aufnahmebereich (B2_{2D}, B2_{3D}) erstreckt,
wobei das Magnetresonanztomographiesystem (1) dazu ausgebildet ist zumindest vor einer Erfassung der zweiten Magnetresonanzrohdaten (RD2) eine magnetische Feldverteilung und eine magnetische Feldverteilung der Gradienten-Nichtlinearität zumindest im zweiten Aufnahmebereich (B2_{2D}, B2_{3D}) zu ermitteln.

11. Computerprogrammprodukt mit einem Computerprogramm, welches direkt in eine Speichereinrichtung einer Steuereinrichtung (13) eines Magnetresonanztomographiesystems (1) ladbar ist, mit Programmabschnitten, um alle Schritte des Verfahrens nach einem der Ansprüche 1 bis 9 auszuführen, wenn das Computerprogramm in der Steuereinrichtung (13) des Magnetresonanztomographiesystems (1) ausgeführt wird.

12. Computerlesbares Medium, auf welchem das Computerprogrammprodukt nach Anspruch 11 gespeichert ist.

## Claims

1. Method for generating magnetic resonance recordings of an examination object (O) in a magnetic resonance tomography device (2), having the following steps:
- acquisition of magnetic resonance raw data (RD1, RD2),
wherein first magnetic resonance raw data (RD1) is acquired in a first recording region (B1_{2D}, B1_{3D}) inside a homogeneity volume (SHV) of the magnetic resonance tomography device (2) in a k-space and second magnetic resonance raw data (RD2) is acquired in a second recording region (B2_{2D}, B2_{3D}) outside the homogeneity volume (SHV) of the magnetic resonance tomography device (2) using a signal encoding function which is not present in the form of a conventional Fourier integral and is therefore not acquired in a k-space,
- reconstruction of first image data (BD1) on the basis of the first magnetic resonance raw data (RD1) and reconstruction of second image data (BD2) on the basis of the second magnetic resonance raw data (RD2), wherein
- the first image data (BD1) is reconstructed on the basis of the first magnetic resonance raw data (RD1) by means of a Fourier transform (FFT) and
- the second image data (BD2) is reconstructed on the basis of the second magnetic resonance raw data (RD2) by a signal model-based image reconstruction method (SMR)
- combination of the first image data (BD1) and the second image data (BD2) to form combination image data (BDK) which covers a region which extends in the first recording region (B1_{2D}, B1_{3D}) and the second recording region (B2_{2D}, B2_{3D}),
wherein at least before an acquisition of the second magnetic resonance raw data (RD2) a magnetic field distribution and a magnetic field distribution of the gradient non-linearity is determined at least in the second recording region (B2_{2D}, B2_{3D}).

2. Method according to claim 1, wherein image points of the first image data (BD1) are reconstructed such that they are depicted on a first isotropic grid (25), and image points of the second image data (BD2), following a reconstruction by means of a regridding method, are depicted on an isotropic grid which is adjusted to the first isotropic grid (25).

3. Method according to one of the preceding claims, wherein the second magnetic resonance raw data (RD2) is recorded on iso-frequency contours (IR) and/or iso-frequency surfaces (IO) of a basic magnetic field of the magnetic resonance tomography device (2).

4. Method according to one of the preceding claims, wherein the first magnetic resonance raw data (RD1) and/or the second magnetic resonance raw data (RD2) comprises slice raw data.

5. Method according to one of the preceding claims, wherein the first magnetic resonance raw data (RD1) and/or the second magnetic resonance raw data (RD2) comprises volume raw data.

6. Method according to one of the preceding claims, wherein an MR gradient echo signal and/or an MR spin echo signal is generated for acquisition of magnetic resonance raw data (RD1, RD2) .

7. Method according to one of the preceding claims, wherein in order to acquire the second magnetic resonance raw data (RD2), a slice-selective RF excitation pulse (HF1, HF2) is played out, wherein a bipolar gradient pulse (G_{S}1) is played out preferably consistent with the slice-selective RF excitation pulse (HF1, HF2).

8. Method according to one of the preceding claims, wherein in order to acquire the second magnetic resonance raw data (RD2), a non-slice-selective RF excitation pulse (HF1', HF2`) is played out, wherein a phase-encoding pulse (G_{P}1) is played out preferably subsequent to the RF excitation pulse (HF1', HF2`).

9. Method according to one of the preceding claims, wherein the determination of the magnetic field distribution and the magnetic field distribution of the gradient non-linearity is carried out before emission of the RF excitation pulse (HF1, HF1', HF2, HF2`) and/or before playing-out of the gradient pulse (G_{S}1) and/or of the phase-encoding pulse (G_{P}1).

10. Magnetic resonance tomography system (1) comprising a magnetic resonance tomography device and a control device (13) for controlling the magnetic resonance tomography device (2), wherein the control device (13) comprises at least:
- an acquisition control unit (14) which is configured to prompt the magnetic resonance tomography device to acquire magnetic resonance raw data (RD1, RD2), wherein first magnetic resonance raw data (RD1) is acquired in a first recording region (B1_{2D}, B1_{3D}) inside a homogeneity volume (SHV) of the magnetic resonance tomography device (2) in a k-space and second magnetic resonance raw data (RD2) is acquired in a second recording region (B2_{2D}, B2_{3D}) outside the homogeneity volume (SHV) of the magnetic resonance tomography device (2) using a signal encoding function which is not present in the form of a conventional Fourier integral and is therefore not acquired in a k-space,
- an image reconstruction unit (20) which is configured to reconstruct first image data (BD1) on the basis of the first magnetic resonance raw data (RD1) and second image data (BD2) on the basis of the second magnetic resonance raw data (RD2), wherein
- the first image data (BD1) is reconstructed on the basis of the first magnetic resonance raw data (RD1) by means of a Fourier transform (FFT) and
- the second image data (BD2) is reconstructed on the basis of the second magnetic resonance raw data (RD2) by a signal model-based image reconstruction method (SMR)
- a combination unit (18) which is configured to combine the first image data (BD1) and the second image data (BD2) to form combination image data (BDK), which covers a region which extends in the first recording region (B1_{2D}, B1_{3D}) and the second recording region (B2_{2D}, B2_{3D}),
wherein the magnetic resonance tomography system (1) is embodied, at least before an acquisition of the second magnetic resonance raw data (RD2), to determine a magnetic field distribution and a magnetic field distribution of the gradient non-linearity at least in the second recording region (B2_{2D}, B2_{3D}) .

11. Computer program product having a computer program, which can be loaded directly into a storage device of a control device (13) of a magnetic resonance tomography system (1), having program segments in order to carry out all steps of the method according to one of claims 1 to 9 when the computer program is run in the control device (13) of the magnetic resonance tomography system (1).

12. Computer-readable medium on which the computer program product according to claim 11 is stored.

## Revendications

1. Procédé de production d'enregistrements de résonnance magnétique d'un objet (O) en examen dans un appareil (2) de tomodensitométrie par résonnance magnétique, comportant les stades suivants :
- acquisition de données (RD1, RD2) brutes de résonnance magnétique, dans lequel on acquiert, dans un espace k, des premières données (RD1) brutes de résonnance magnétique dans une première partie (B1_{2D}, B1_{3D}) d'enregistrement au sein d'un volume (SHV) d'homogénéité de l'appareil (2) de tomodensitométrie par résonnance magnétique et, dans une deuxième partie (B2_{2D}, B2_{3D}) d'enregistrement à l'extérieur du volume (SHV) d'homogénéité de l'appareil (2) de tomodensitométrie par résonnance magnétique, en utilisant une fonction de codage du signal, qui ne se présente pas sous la forme d'une intégrale de Fourier habituelle, on n'acquiert donc pas, dans un espace k, des deuxièmes données (RD2) brutes de résonnance magnétique,
- reconstruction de premières données (BD1) d'image, sur la base des premières données (RD1) brutes de résonnance magnétique, et reconstruction de deuxièmes données (BD2) d'image, sur la base des deuxièmes données (RD2) brutes de résonnance magnétique, dans lequel
- on reconstruit, au moyen d'une transformation (FFT) de Fourier, les premières données (BD1) d'image en se fondant sur les premières données (RD1) brutes de résonnance magnétique, et
- on reconstruit les deuxièmes données (BD2) d'image, sur la base des deuxièmes données (RD2) brutes de résonnance magnétique par un procédé (SMR) de reconstruction d'image reposant sur un modèle de signal,
- combinaison des premières données (BD1) d'image et des deuxièmes données (BD2) d'image en des données (BDK) d'image de combinaison, qui recouvrent une partie, qui s'étend dans la première partie (B1_{2D}, B1_{3D}) d'enregistrement et dans la deuxième partie (B2_{2D}, B2_{3D}) d'enregistrement,
dans lequel, au moins avant une saisie des deuxièmes données (RD2) brutes de résonnance magnétique, on détermine une répartition du champ magnétique et une répartition du champ de la non linéarité de gradients au moins dans la deuxième partie (B2_{2D}, B2_{3D}) d'enregistrement.

2. Procédé suivant la revendication 1, dans lequel on reconstruit des points image des premières données (BD1) d'image, de manière à ce qu'elles soient représentées sur un premier quadrillage (25) isotrope, et on représente des points image des deuxièmes données (BD2) d'image suivant une reconstruction, au moyen d'un procédé de requadrillage, sur un quadrillage isotrope, qui est adapté au premier quadrillage (25) isotrope.

3. Procédé suivant l'une des revendications précédentes, dans lequel on enregistre les deuxièmes données (RD2) brutes de résonnance magnétique sur des contours (IR) de fréquence et/ou des surfaces (IO) de fréquence ISO d'un champ magnétique de base de l'appareil (2) de tomodensitométrie par résonnance magnétique.

4. Procédé suivant l'une des revendications précédentes, dans lequel les premières données (RD1) brutes de résonnance magnétique et/ou les deuxièmes données (RD2) brutes de résonnance magnétique comprennent des données brutes de couche.

5. Procédé suivant l'une des revendications précédentes, dans lequel les premières données (RD1) brutes de résonnance magnétique et/ou les deuxièmes données (RD2) brutes de résonnance magnétique comprennent des données brutes de volume.

6. Procédé suivant l'une des revendications précédentes, dans lequel, pour l'acquisition de données (RD1, RD2) brutes de résonnance magnétique, on produit un signal d'écho de gradient RM et/ou un signal d'écho de spin RM.

7. Procédé suivant l'une des revendications précédentes, dans lequel, pour la détection des deuxièmes données (RD2) brutes de résonnance magnétique, on fait jouer une impulsion (HF1, HF2) d'excitation HF sélective en couche, dans lequel, de préférence, on fait jouer une impulsion (G_{S}1) de gradient bipolaire s'adaptant à une impulsion (HF1, HF2) d'excitation HF sélective en couche.

8. Procédé suivant l'une des revendications précédentes, dans lequel, pour la saisie des deuxièmes données (RD2) brutes de résonnance magnétique, on fait jouer une impulsion (HF1', HF2") d'excitation HF non sélective en couche, dans lequel, de préférence on fait jouer, après l'impulsion (HF1', HF2") d'excitation HF, une impulsion (G_{P}1) de codage de phase.

9. Procédé suivant l'une des revendications précédentes, dans lequel la détermination de la répartition du champ magnétique et de la répartition du champ magnétique de la non linéarité de gradients a lieu avant l'émission de l'impulsion (HF1, HF1', HF2, HF2") d'excitation HF et/ou avant de faire jouer l'impulsion (G_{S}1) de gradient et/ou l'impulsion (G_{P}1) de codage de phase.

10. Système (1) de tomodensitométrie par résonnance magnétique comprenant un appareil de tomodensitométrie par résonnance magnétique et un dispositif (13) de commande pour la commande de l'appareil (2) de tomodensitométrie par résonnance magnétique, dans lequel le dispositif (13) de commande comprend au moins :
- une unité (14) d'acquisition, qui est conçue pour faire que l'appareil de tomodensitométrie par résonnance magnétique acquiert des données (RD1, RD2) brutes de résonnance magnétique, dans lequel on acquiert, dans un espace k, des premières données (RD1) brutes de résonnance magnétique dans une première partie (B1_{2D}, B1_{3D}) d'enregistrement au sein d'un volume (SHV) d'homogénéité de l'appareil (2) de tomodensitométrie par résonnance magnétique et, dans une deuxième partie (B2_{2D}, B2_{3D}) d'enregistrement à l'extérieur du volume (SHV) d'homogénéité de l'appareil (2) de tomodensitométrie par résonnance magnétique, en utilisant une fonction de codage du signal, qui ne se présente pas sous la forme d'une intégrale de Fourier habituelle, on n'acquiert donc pas, dans un espace k, des deuxièmes données (RD2) brutes de résonnance magnétique,
- une unité (20) de reconstruction d'image, qui est conçue pour reconstruire des premières données (BD1) d'image sur la base des premières données (RD1) brutes de résonnance magnétique et de deuxièmes données (BD2) d'image sur la base des deuxièmes données (RD2) brutes de résonnance magnétique,
dans lequel
- on reconstruit, au moyen d'une transformation (FFT) de Fourier, des premières données (BD1) d'image en se fondant sur les premières données (RD1) brutes de résonnance magnétique, et
- on reconstruit les deuxièmes données (BD2) d'image, sur la base des deuxièmes données (RD2) brutes de résonnance magnétique par un procédé (SMR) de reconstruction d'image reposant sur un modèle de signal,
- une unité (18) de combinaison, qui est conçue pour combiner les premières données (BD1) d'image et les deuxièmes données (BD2) d'image en des données (BDK) d'image de combinaison, qui recouvrent une partie, qui s'étend dans la première partie (B1_{2D}, B1_{3D}) d'enregistrement et dans la deuxième partie (B2_{2D}, B2_{3D}) d'enregistrement,
dans lequel le système (1) de tomodensitométrie par résonnance magnétique est constitué pour déterminer, avant une saisie des deuxièmes données (RD2) brutes de résonnance magnétique, une répartition du champ magnétique et une répartition du champ de la non linéarité de gradients au moins dans la deuxième partie (B2_{2D}, B2_{3D}) d'enregistrement.

11. Produit de programme d'ordinateur comprenant un programme d'ordinateur, qui peut être chargé directement dans un dispositif de mémoire d'un dispositif (13) de commande d'un système (1) de tomodensitométrie par résonnance magnétique ayant des parties de programme pour exécuter tous les stades du procédé suivant l'une des revendications 1 à 9, lorsque le programme d'ordinateur est exécuté dans le dispositif (13) de commande du système (1) de tomodensitométrie par résonnance magnétique.

12. Support, déchiffrable par ordinateur, sur lequel est mis en mémoire le produit de programme d'ordinateur suivant la revendication 11.
